Europäisches Patentamt

European Patent Office

Office européen des brevets

(11) Veröffentlichungsnummer: **0 278 378 B1**

(12) **EUROPÄISCHE PATENTSCHRIFT**

(45) Veröffentlichungstag der Patentschrift: **08.01.92**

(51) Int. Cl.⁵: **C07C 50/24**, C07C 46/06

(21) Anmeldenummer: **88101534.1**

(22) Anmeldetag: **03.02.88**

(54) Verfahren zur Herstellung von Tetrachlor-1,4-benzochinon hoher Reinheit.

(30) Priorität: **06.02.87 DE 3703566**
**06.03.87 DE 3707148**

(43) Veröffentlichungstag der Anmeldung:
**17.08.88 Patentblatt 88/33**

(45) Bekanntmachung des Hinweises auf die
Patenterteilung:
**08.01.92 Patentblatt 92/02**

(84) Benannte Vertragsstaaten:
**BE CH DE ES FR GB IT LI NL SE**

(56) Entgegenhaltungen:
EP-A- 0 220 135
AT-B- 127 813
DD-A- 9 531
DE-B- 1 178 838

HOUBEN-WEYL, METHODEN DER ORGANISCHEN CHEMIE, Band VII/3a, 4. Auflage, 1977, Seiten 148 - 151, Stuttgart, Georg Thieme Verlag

(73) Patentinhaber: **HOECHST AKTIENGESELLSCHAFT**
**Postfach 80 03 20**
**W-6230 Frankfurt am Main 80(DE)**

(72) Erfinder: **Arndt, Otto, Dr.**
**Frankfurter Strasse 38**
**W-6238 Hofheim am Taunus(DE)**
Erfinder: **Papenfuhs, Theodor, Dr.**
**Heinrich-Bleicher-Strasse 40**
**W-6000 Frankfurt am Main 50(DE)**

**Beschreibung**

Die vorliegende Erfindung betrifft ein Verfahren zur Herstellung von Tetrachlor-1,4-benzochinon (nachstehend als "Chloranil" bezeichnet) hoher Reinheit aus Hydrochinon.

Die Herstellung von Chloranil aus Hydrochinon (1,4-Dihydroxybenzol) oder 1,4-Benzochinon bzw. chloriertem 1,4-Benzochinon ist an sich nach folgenden in der Literatur beschriebenen Verfahren bekannt:

1. Chlorieren von Hydrochinon mit konzentrierter Salzsäure und konzentriertem Wasserstoffperoxid in Gegenwart von Magnesiumchlorid (DE-OS 2 645 114);

2. Einwirkung von Chlor auf Hydrochinon in siedender konzentrierter Salzsäure (Chemiker Zeitung 56 (1932), Seite 569);

3. Einwirkung von Salzsäure und Salpetersäure (Königswasser) auf Hydrochinon (J. Chem. Soc. Japan 63 (1942), Seite 1441);

4. Reaktionen von Antimon(V)-chlorid mit Hydrochinon (Chemiker Zeitung 104 (1980) Nr. 1, Seiten 13 und 14;

5. Einwirkung von Chlorwasserstoff, Luft und Metallsalzen auf Hydrochinon (DDR-Patentschrift Nr. 29 292);

6. Reaktion von Trichlor-1,4-Benzochinon mit Chlor bei Gegenwart von Jod und Wasser (Liebigs Annalen der Chemie, Supplementband 6 (1867), 213);

7. Behandlung eines Gemenges aus Trichlor-1,4-benzochinon und Tetrachlor-1,4-benzochinon mit Chlorwasserstoff in Eisessig und anschließende Einwirkung von konzentrierter Salpetersäure (Beilstein 7 637);

8. Einwirkung von konzentrierter Salzsäure und 35 %igem Wasserstoffperoxid auf 1,4-Benzochinon (Ann. Chimica applic. 22 (1932) 602;

9. Einleiten von Chlor in mit Chromtrioxid versetzte Lösung von Hydrochinon in wäßriger Salzsäure (Naugatuck Chem. Comp., USA, DRP 594 520, Friedländer 20, 2047, US-Patent 1 918 328).

Diese bekannten Verfahren haben jedoch folgende Nachteile:

Ad 1:    Es ist ein sehr großer Überschuß von Salzsäure (96fach molare Menge) erforderlich und es resultiert eine große Salzbelastung durch Zugabe von Magnesiumchlorid in der 7,4fach molaren Menge. Hinzu kommt, daß die angegebene zeitliche Temperaturführung bei der Zudosierung des Wasserstoffperoxids wegen der hohen Wärmetönung nicht eingehalten werden kann. Die Nacharbeitung dieses Verfahrens mit nur 30fach molarer Menge Salzsäure, 3fach molarer Menge Magnesiumchlorid und einer geeigneteren Temperaturführung ergab ein qualitativ schlechtes Chloranil (Fp. 215 - 220° C, Ausbeute 95 % der Theorie) mit Verunreinigungen an Trichlor-1,4-benzochinon und Tetrachlorhydrochinon.

Ad 2:    Das Eingasen von elementarem Chlor in siedende konzentrierte Salzsäure führt zu starkem Abgasen von elementarem Chlor in die Chlorwasserstoff-Brüden und macht die Anwendung eines großen Chlorüberschusses notwendig.

Die in der genannten Literaturstelle nicht angegebene Möglichkeit des Arbeitens unter Druck (im Autoklav) erfordert erhöhten technischen und sicherheitstechnischen Aufwand (voll emaillierte Armaturen, Ventile und Leitungsanschlüsse).

Die dort angegebene Menge von 37 %iger Salzsäure (28fach molare Menge) ist sehr hoch, reicht offenbar jedoch nicht aus, um ein bei 25° C rührbares Reaktionsgemisch zu erhalten. Das Gemisch erstarrt unter diesen Bedingungen unter Bildung von Tetrachlorhydrochinon.

Ad 3:    Bei diesem Verfahren wird eine etwa 25fach molare Menge Mineralsäure, zusammengesetzt aus 15fach molarer Menge Salzsäure und etwa 10fach molarer Menge Salpetersäure, eingesetzt. Die Ausbeute beträgt nur etwa 45 - 65 % der Theorie (Fp. 280° C). Über die Entsorgung des Säureüberschusses (besonders der Stickoxide) gibt es keines Hinweise.

Ad 4:    Die Verwendung von Antimon ist toxikologisch bedenklich und erfordert eine aufwendige Rückgewinnung. Außerdem soll bei diesem Verfahren Phosgen entstehen.

Ad 5:    In der genannten Patentschrift wird selbst auf den hohen Aufwand an Hilfsmitteln und die niedrige Ausbeute hingewiesen. Außerdem verbraucht die Wasserdampfdestillation viel Energie.

Ad 6:    Die Verwendung von Jod erschwert die Regeneration der Salzsäure zur Wiederverwertung.

Ad 7:    Der stufenweise Aufbau aus Chinon (hier: Trichlor-1,4-benzochinon) und Salzsäure mit anschließender Oxidation (hier: Salpetersäure) des chlorierten Hydrochinons (hier: Tetrachlorhydrochinon) zum Chinon mit den notwendigen zwischenstuflichen Reinigungsoperationen ist ein äußerst zeitraubendes Verfahren und ist für ein Betriebsverfahren ungeeignet.

Ad 8: Bei dem Verfahren wird zunächst mit konzentrierter Salzsäure (22 Bé = 37 %) (11fach molare Menge) 20 Stunden, anschließend mit Wasserstoffperoxid 35 % unterhalb von 60°C 12 Stunden behandelt. Man erhält zwar das Chloranil in einer hohen Ausbeute (Fp. 289 bis 290°C), durch die geringe Raum-Zeit-Ausbeute wird die Herstellung jedoch sehr teuer.

Ad 9: Bei dem Verfahren wird Hydrochinon in Salzsäure mit Chrom(VI)-oxid (20 g/Mol) zum Chinhydron oxidiert und dieses dann zunächst bei 25°C, schließlich in der Hitze mit elementarem Chlor zum Chloranil chloriert. Bei den heutigen Anforderungen des Umweltschutzes läßt sich die Anwendung von Chrom(VI)-oxid wirtschaftlich nicht mehr vertreten.

Der vorstehend gegebene Überblick über den Stand der Technik zeigt, daß die bekannten Verfahren im allgemeinen einen hohen Überschuß an Salzsäure, teilweise sogar umweltbelastende Hilfsstoffe und außergewöhnliche Oxidationsmittel sowie lange Reaktionszeiten benötigen.

Es wurde nun überraschenderweise gefunden, daß sich der Materialeinsatz (insbesondere an Salzsäure und Chlorierungsmittel) und der Zeitaufwand erheblich verringern lassen, und sich ferner ein Produkt hoher Reinheit erzielen läßt, wenn man Chlorierung und Oxidation zeitlich und temperaturmäßig so gewichtet, daß bis zur Einführung von 2 Chloratomen eine Oxidation zu Chinhydron oder Chinon vermieden wird, wodurch die Einführung des dritten Chloratoms zeitgleich mit der Aufoxidation zum Trichlorbenzochinon erfolgt.

Gegenstand der Erfindung ist somit ein verbessertes Verfahren zur Herstellung von Tetrachlor-1,4-benzochinon hoher Reinheit, indem man auf 1 Mol Hydrochinon (Molgewicht 110,1) in der 6- bis 7fach molaren Menge 30 bis 37 %iger Salzsäure die 1,9- bis 2,1fach, vorzugsweise 2fach molare Menge gasförmigen Chlors bei Temperaturen von 5 bis 15°C einwirken läßt, dann die angefallene, im wesentlichen das 2,5-Dichlorhydrochinon enthaltende Suspension mit 2 bis 3 Gewichtsteilen Wasser, bezogen auf Gewichtsteile eingesetztes Hydrochinon, verdünnt und zunächst auf eine Temperatur von 20 bis 30°C erwärmt, anschließend unter Luftatmosphäre und unter Erhöhung der Temperatur auf 90 bis 100°C die 1,9- bis 2,1fach, vorzugsweise 2fach molare Menge Chlorgas einleitet, unter gleichzeitiger Verdünnung mit 3,5- bis 4,5-, vorzugsweise 4 Gewichtsteilen Wasser, bezogen auf Gewichtsteile eingesetztes Hydrochinon, und schließlich die 1- bis 2fach molare Menge gasförmigen Chlors, bezogen auf eingesetztes Hydrochinon, bei Temperaturen von 100 bis 115°C einleitet.

Der Grund für die verbesserte Verfahrensführung ist die Ausnutzung der höheren Löslichkeiten der 1- bis 3fach chlorierten Hydrochinone in der konzentrierten Salzsäure im Vergleich zu den entsprechend 1- bis 3fach chlorierten 1,4-Benzochinonen.

Abweichungen vom erfindungsgemäßen Verfahren dokumentieren sich in erhöhtem Verbrauch von Chlorierungsmittel und schlechter Qualität des Chloranils (erhöhter Gehalt an Tetrachlorhydrochinon, 2,3- und 2,5-Dichlor-1,4-benzochinon, Trichlor-1,4-Benzochinon und unbekannten Nebenkomponenten) (erfaßt durch HPLC = high performance liquid chromatography und HPTLC = high performance thin layer chromatography).

Der durch die höheren Löslichkeiten der 1- bis 3fach chlorierten Hydrochinone bedingte raschere Umsatz mit dem Chlorierungsmittel wird durch Verwendung von säure- und chlorstabilen oberflächenaktiven Hilfsmitteln noch unterstützt, wobei diese zudem ein mögliches Schäumen der Reaktionsmischung wirkungsvoll unterbinden. Als derartiges Hilfsmittel eignet sich sekundäres Alkansulfonat oder ein Entschäumer auf Basis fluorierter Anionen. Die oberflächenaktiven Hilfsmittel werden zweckmäßigerweise in einer Menge von etwa 5 bis 15 Millimol pro Mol eingesetztem Hydrochinon angewendet.

Von großer Bedeutung ist auch die Konzentration der Salzsäure, insbesondere zu Beginn der Chlorierung. Bei Startkonzentrationen von Salzsäure unterhalb von 30 % werden stark gefärbte Reaktionsmischungen erhalten, die schließlich ein Chloranil schlechter Qualität ergeben. Durch eine ausreichend hohe Startkonzentration der Salzsäure wird das Reduktionspotential der Hydrochinone so weit herabgesetzt, daß sie im Sinn der Erfindung bis zur Einführung des dritten Chloratoms von einer zu frühzeitigen Oxidation zum 1,4-Benzochinon bewahrt bleiben.

Die Salzsäure-Konzentration nimmt während des Umsatzes zu, weshalb es erforderlich ist, während der Reaktion mit Wasser zu verdünnen, was zweckmäßigerweise durch ein bestimmtes Temperatur-, Zeit- und Zudosierungsprogramm erfolgt. Durch die Verdünnung kann die anzuwendende Menge an Salzsäure erheblich gesenkt werden, bleibt das Reaktionsgemisch rührbar und wird eine Emission von elementarem Chlor vor Beendigung der Umsetzung verhindert. Ein Durchleiten von Luft ab 50°C fördert die Aufoxidation zum chlorierten p-Benzochinon.

Das erfindungsgemäße Verfahren ist im Vergleich zum Stand der Technik ökonomisch und ökologisch vorteilhaft. Die Mutterlaugen werden auf 20 %ige Salzsäure destillativ regeneriert. Die Regeneratsäuren sind farblos, enthalten höchstens Spuren organischen Kohlenstoffs und können an anderer Stelle der Produktion wieder eingesetzt werden. Außer dem Waschfiltrat und einem im wesentlichen aus sekundärem Alkansulfat bestehenden Destillationsrückstand fallen keine weiteren zu entsorgenden Fabrikationsrückstände an.

Das erfindungsgemäß hergestellte Chloranil ist von hoher Reinheit, was sich am Schmelzpunkt (281 -282° C) und daran, daß es kein Tetrachlorhydrochinon enthält, zeigt.

C-Gehalt: 29,3 - 29,7 % (theoretisch 29,31 %)
Cl-Gehalt: 57,3 - 57,6 % (theoretisch 57,67 %)
Reingehalt (titanometrisch) = 100,0 %

Nachstehend sei noch eine bevorzugte Ausführung des erfindungsgemäßen Verfahrens angegeben, wobei Teile Gewichtsteile sind:

1 Teil Hydrochinon wird in 6,3 Teilen Salzsäure 31 % (entspricht der 6fach molaren Menge, bezogen auf das eingesetzte Hydrochinon) in Gegenwart von ca. 0,025 Teilen sekundärem Alkansulfonat bei 10° C zunächst mit nur ca. 1,3 Teilen Chlorgas (2fach molare Menge) umgesetzt. Die Reaktion ist stark exotherm. Es bildet sich eine dicke, hellbeigefarbene Suspension. Die Suspension wird dann mit ca. 2 Teilen (bezogen auf Hydrochinon) Wasser verdünnt und auf 25° C erwärmt. Das bis zu diesem Zeitpunkt gebildete Reaktionsprodukt hat folgende Zusammensetzung:

64 Mol-% 2,5-Dichlorhydrochinon,
23 Mol-% 2,3-Dichlorhydrochinon und
13 Mol-% 2-Chlorhydrochinon,

Unter weiterem Aufheizen bis auf 95° C, vorteilhafterweise unter Durchleiten von Luft, werden nochmals ca. 1,3 Teile Chlor (2fach molare Menge, bezogen auf eingesetztes Hydrochinon) eingegast. Gleichzeitig wird mit ca. 4 Teilen (bezogen auf eingesetztes Hydrochinon) Wasser verdünnt. Die Suspension ändert ihre Farbe nach Hellgelb.

Das letzte noch nötige Mol Chlor wird bei 105° C eingegast unter gleichzeitigem Zusatz von ca. 4 Teilen (bezogen auf eingesetztes Hydrochinon) Wasser und Durchleiten von Luft. Insgesamt werden ca. 5,3 Mol Chlor eingegast. Erst gegen Ende des Chlorzusatzes entwickelt sich Abgas (ca. 20 Mol-%, bezogen auf Hydrochinon), entsprechend dem eingesetzten Chlorüberschuß. Das Abgas enthält keinen Chlorwasserstoff. Nach Filtrieren, und Waschen erhält man reines Chloranil in einer Ausbeute von 98 % der Theorie, bezogen auf eingesetztes Hydrochinon.

Die ca. 20 %ige Mutterlauge wird auf eine 20 %ige Destillatsalzsäure aufgearbeitet (organischer Kohlenstoff = 69 mg/1).

Als Fabrikationsrückstände fallen nur das eingesetzte sekundäre Alkansulfonat (Destillationsrückstand bei Salzsäure-Regeneration) und das Waschfiltrat an. Letzteres kann biologisch gereinigt werden (Rest-CSB = 3,7 kg Sauerstoff ($O_2$) pro to Chloranil).

Chloranil ist ein wertvolles Zwischenprodukt zur Herstellung von Farbstoffen und Schädlingsbekämpfungsmitteln. Ferner wird es als Photochemikalie und Vulkanisationsmittel eingesetzt und dient als Zusatz für Schmiermittel.

Das nachstehende Beispiel und die angegebenen Vergleichsbeispiele dienen zur näheren Erläuterung der Erfindung.

## Beispiel 1

In ein Gemisch aus 353 Teilen Salzsäure 31 % (3,0 Mol), 55,6 Teilen Hydrochinon (0,5 Mol) und 1,4 Teilen sekundärem n-Alkansulfonat werden bei 10° C unter Außenkühlung 73 Teile Chlorgas (1,0 Mol) in 120 Minuten eingegast. Dann rührt man 30 Minuten bei 10° C nach. Anschließend wird die dicke, hellbeigefarbene Suspension mit 100 Teilen Wasser verdünnt und in 60 Minuten auf 25° C erwärmt.

Darauf werden in 210 Minuten erneut 73 Teile Chlorgas (1,0 Mol) eingegast, wobei gleichzeitig 200 Teile Wasser hinzugetropft werden und gleichzeitig auf 95° C aufgeheizt wird. Ab ca. 50° C wird gleichzeitig Luft durchgeleitet bis zum Ende der Reaktion.

In die nunmehr hellgelbe, nicht schäumende Suspension werden in 60 Minuten 18 Teile (0,25 Mol) Chlorgas eingegast, wobei gleichzeitig 40 Teile Wasser hinzugetropft werden und gleichzeitig auf 105° C aufgeheizt wird. Anschließend rührt man 300 Minuten bei 105° C nach, wobei je nach Umsatzfortschritt (das Fortschreiten der Chlorierung und Oxidation wird mit HPTLC verfolgt) noch maximal 26 Teile Chlorgas (0,37 Mol) eingegast werden müssen. Gleichzeitig werden 160 Teile Wasser hinzugetropft (Heizbad 115° C).

Die Chlorierzeit beträgt 13,5 Stunden. Insgesamt werden 190 Teile Chlor (2,7 Mol) eingegast. Nach Beendigung der Umsetzung spült man das restliche in der Reaktoratmosphäre vorhandene Chlorgas in eine Vorlage aus 250 Teilen Wasser und 125 Teilen Natronlauge 33 % (0,10 Mol $Cl_2$). Das Abgas enthält keinen Chlorwasserstoff.

Nach Filtration bei 90 bis 95° C und Waschen mit 500 Teilen Wasser erhält man 121 Teile reines gelbes Chloranil (0,49 Mol).

C-Gehalt: 29,3 - 29,7 % (theoretisch 29,31 %)
Cl-Gehalt: 57,3 - 57,6 % (theoretisch 57,67 %)
Reingehalt (titanometrisch) = 100 %
Schmelzpunkt: 281 - 282° C.

Die Mutterlauge (996 Teile ca. 20 %ige Salzsäure) wird bei Normaldruck auf Rückstand destilliert. Man erhält 960 Teile Salzsäure 20 % (farblos, C-organisch = 96 mg/1) und 5,1 Teile Destillationsrückstand, der sich mit Wasser aus dem Destillationskolben entfernen läßt. Das Waschfiltrat läßt sich biologisch reinigen.

**Beispiel 2** (praxisgerechte Ausführung):

In ein gemisch aus 353 Teilen Salzsäure 31 % (3,0 Mol), 55,6 Teilen Hydrochinon (0,50 Mol), 0,024 Teilen Eisen(III)chlorid und 0,3 Teilen sekundärem n-Alkansulfonat werden bei 10° C unter Außenkühlung und bei Normaldruck (Luft) 73 Teile Chlorgas (1,0 Mol) in 4 Stunden eingegast. Dann rührt man 30 Minuten bei 10° C nach. Anschließend wird die dicke, hellbeigefarbene Suspension mit 400 Teilen Wasser in ca. 2 Minuten verdünnt, wobei sie sich spontan auf 25° C erwärmt. Darauf werden in 4 Stunden 93 Teile Chlorgas (1,3 Mol) eingegast unter gleichzeitigem Aufheizen auf 95° C. Die Suspension wird hellgelb. Es entsteht kein Abgas.

Dann wird die Suspension (HCl-Gehalt = ca. 24%) mit 100 Teilen Trinkwasser verdünnt. In die nunmehr hellgelbe, nicht schäumende Suspension werden in 1 Stunde 14 Teile Chlorgas (0,2 Mol) eingegast unter gleichzeitigem Aufheizen auf 105° C. Das Chlor wird so eingegast, daß gerade kein Abgas entweicht.

Anschließend rührt man 10 Stunden bei 108° C nach, wobei je nach Umsatzfortschritt (das Fortschreiten der Chlorierung und Oxidation wird mit HPTLC verfolgt) noch maximal 45 Teile Chlorgas (0,6 Mol) eingegast werden müssen (Temperatur des Heizbads 130° C). Die dünne, nun zitronengelbe Suspension wird noch 1 Stunde bei 108° C nachgerührt.

Die Chlorierzeit beträgt maximal 20 Stunden. Insgesamt werden 225 Teile Chlor (3,17 Mol) eingegast. Man kühlt den Ansatz auf 90° C unter Überlagerung mit Schutzgas (Stickstoff) ab, wodurch ein Zurücksaugen von Chlor aus der Abgasabsorption verhindert wird und die Atmosphäre über dem Reaktionsgemisch entgiftet wird. Der Abgasstrom wird in der Absorptionslauge aufgenommen (45 Teile = 0,65 Mol).
Umgesetztes Chlor = 180 Teile = 2,54 Mol Chlor (theor. = 2,50 Mol).

Nach Filtration bei 90 - 95° C und Waschen mit 500 Teilen Wasser erhält man 121 Teile reines gelbes Chloranil (0,49 Mol), Fp. 293 - 295° C.
C-Gehalt: 29,6 % (theor. 29,31 %)
Cl-Gehalt: 57,3 % (theor. 57,67 %)
Reingehalt (titanometrisch) = 100 %
2,3,5,6-Tetrachlorhydrochinon nicht nachweisbar (HPTLC)
2,3,5-Trichlorbenzochinon < 0,5 % (HPLC)

Hexachlorbenzol nicht nachweisbar (GC/MS)
Die Mutterlauge (970 Teile) ist eine ca. 20 %ige Salzsäure (5,32 Mol HCl).

**Beispeil 3** (Recycling Mutterlauge)

Man verfährt wie in Beispiel 2 beschrieben, wobei jedoch statt der 31 %igen Salzsäure 547 Teile Mutterlauge (aus Beispiel 2, entsprechend 3,0 Mol HCl) vorgelegt werden. Nach dem Eingasen von 73 Teilen Chlorgas bei 10° C in 4 Stunden wird jedoch nur mit 240 Teilen Wasser verdünnt.

Man erhält Chloranil in gleicher Ausbeute und Qualität wie bei Beispiel 2.

Das Recycling der Mutterlauge läßt sich bis ca. 20 mal wiederholen.

Man erhält Chloranil in gleicher Ausbeute und Qualität, wenn man beachtet, daß die Hilfsstoffe (Tensid und FeCl₃) in ihrer Konzentration durch entsprechende Nachdotierung erhalten bleiben und die einzusetzende Mutterlauge immer auf den Gehalt von 20 % HCl korrigiert wird.

**Vergleichsbeispiel 1**

Man verfährt wie in Beispiel 1 beschrieben, jedoch mit dem Unterschied, daß man den Ansatz statt mit Wasser mit 31 %iger Salzsäure (587 Teile) verdünnt. Schon ab 50° C setzt die Bildung eines Abgases ein.

Der Fortschritt des Umsatzes wird mit HPTLC verfolgt.

Nach Eingasen von 146 Teilen (2,1 Mol) Chlorgas, einer Zeitdauer von 7,5 Stunden, wird auf dem Temperaturniveau von 95° C eine dicke, hellbeigefarbene, vorwiegend aus Tetrachlorhydrochinon bestehende Suspension erhalten. Im Abgas wurden 21 Teile Chlor gefunden. Nach Eingasen von weiteren 84 Teilen (1,2 Mol) Chlorgas und einer Zeitdauer von weiteren 8 Stunden wird auf dem Temperaturniveau von 105° C eine dünne, hellgelbe Suspension erhalten, aus welcher unter den Aufarbeitungsbedingungen gemäß Beispiel 106,4 Teile Chloranil mit einem Schmelzpunkt von 255 bis 260° C isoliert werden. Das Chloranil enthält noch deutlich Mono-, Di- und Tetrachlorhydrochinon.

Im Abgas werden 119 Teile Chlorwasserstoff und 32 Teile Chlor (0,45 Mol Cl₂) gefunden. Stärkere Salzsäurekonzentrationen im Chlorierungsgemisch verschieben allzu stark die Oxidation des Hydrochinons zum 1,4-Benzochinon nach höherem Chlor-Verbrauch und höherer Temperatur. Die Verdünnung mit Wasser ist also ein wichtiger Faktor.

**Vergleichsbeispiel 2**

Man verfährt wie im Beispiel 1 beschrieben, jedoch mit dem Unterschied, daß das dort eingangs genannte Gemisch statt 353 Teilen Salzsäure 31 % (3,0 Mol) 228 Teile Salzsäure 20 % (1,25 Mol) enthält.

Beim Eingasen des Chlors bei 10°C wird die Hydrochinon-Suspension sofort schwarz. Nach Beenden der Chlorierung mit 73 Teilen Chlorgas auf dem Temperaturniveau von 10°C wird eine dicke, gelbgrünliche Suspension erhalten, die sich beim Aufheizen auf 50°C nach Hellbraun verfärbt.

Die Fortsetzung der Chlorierung und Aufarbeitung gemäß Beispiel ergibt 122 Teile Chloranil vom Schmelzpunkt 242 bis 246°C.
Reingehalt (titanometrisch) = 76,3 %
C : Cl = 6,0 : 3,82

Das Produkt ist stark mit Monochlorhydrochinon verunreinigt.

### Vergleichsbeispiel 3

Man verfährt wie in Beispiel 1 beschrieben, jedoch unter Ausschluß von Luft (z.B. unter Stickstoff). Man erhält 121 Teile reines Tetrachlorhydrochinon als weißes Pulver mit Schmelzpunkt 230 bis 235°C. Das Abgas enthält nunmehr entsprechend mehr Chlor (0,50 Mol $Cl_2$).

### Patentansprüche

1. Verfahren zur Herstellung von Tetrachlor-1,4-benzochinon hoher Reinheit durch Einwirkung von Chlor und Salzsäure auf Hydrochinon, dadurch gekennzeichnet, daß man auf 1 Mol Hydrochinon (Molgewicht 110,1) in der 6- bis 7fach molaren Menge 30 bis 37 %iger Salzsäure die 1,9- bis 2,1fach molare Menge gasförmigen Chlors bei Temperaturen von 5 bis 15°C einwirken läßt, dann die angefallene, im wesentlichen das 2,5-Dichlorhydrochinon enthaltende Suspension mit 2 bis 3 Gewichtsteilen Wasser, bezogen auf Gewichtsteile eingesetztes Hydrochinon, verdünnt und zunächst auf eine Temperatur von 20 bis 30°C erwärmt, anschließend unter Luftatmosphäre und unter Erhöhung der Temperatur auf 90 bis 100°C die 1,9- bis 2,1fach molare Menge Chlorgas einleitet unter gleichzeitiger Verdünnung mit 3,5 bis 4,5 Gewichtsteilen Wasser, bezogen auf Gewichtsteile eingesetztes Hydrochinon, und schließlich die 1- bis 2fach molare Menge gasförmigen Chlors, bezogen auf eingesetztes Hydrochinon, bei Temperaturen von 100 bis 115°C einleitet.

2. Verfahren nach Anspruch 1, dadurch gekennzeichnet, daß man in Gegenwart eines säure- und chlorstabilen oberflächenaktiven Mittels arbeitet.

### Claims

1. A process for the production of high-purity tetrachloro-1,4-benzoquinone by the action of chlorine and hydrochloric acid on hydroquinone, which comprises causing 1.9 to 2.1 times the molar quantity of gaseous chlorine to act at temperatures from 5 to 15°C on 1 mol of hydroquinone (molecular weight 110.1) in 6 to 7 times the molar quantity of 30 to 37 % hydrochloric acid, then diluting the resulting suspension, essentially containing 2,5-dichlorohydroquinone, with 2 to 3 parts by weight of water, relative to parts by weight of hydroquinone employed, and initially warming to a temperature from 20 to 30°C, subsequently introducing 1.9 to 2.1 times the molar quantity of chlorine gas in an air atmosphere while raising the temperature to 90 - 100°C with simultaneous dilution with 3.5 to 4.5 parts by weight of water, relative to parts by weight of hydroquinone employed, and finally introducing 1 to 2 times the molar quantity of gaseous chlorine, relative to hydroquinone employed, at temperatures from 100 to 115°C.

2. The process as claimed in claim 1, which comprises operation in the presence of a surface-active agent which is stable to acid and chlorine.

### Revendications

1. Procédé pour préparer la tétrachlorobenzoquinone-1,4 à l'état très pur par action de chlore et d'acide chlorhydrique sur l'hydroquinone, procédé caractérisé en ce qu'on fait agir sur 1 mol d'hydroquinone (masse moléculaire : 110,1), dans 6 à 7 fois la quantité molaire d'acide chlorhydrique à 30-37%, une quantité de chlore gazeux représentant de 1,9 à 2,1 fois la quantité molaire, à des températures de 5 à 15°C, puis on dilue la suspension obtenue, contenant essentiellement la dichloro-2,5 hydroquinone, avec de 2 à 3 parties en poids d'eau, par rapport aux parties en poids d'hydroquinone mises en jeu, et on chauffe d'abord à une température de 20 à 30°C, après quoi on introduit, sous une atmosphère d'air et tout en élevant la température à 90-100°C, une quantité de chlore gazeux représentant de 1,9 à 2,1 fois la quantité molaire, en même temps qu'on dilue avec de 3,5 à 4,5 parties en poids d'eau, relativement aux parties en poids d'hydroquinone mises en jeu, et enfin on introduit de 1 à 2 fois la quantité molaire de chlore gazeux, relativement à l'hydroquinone mise en jeu, à des températures de 100 à 115°C.

2. Procédé selon la revendication 1 caractérisé en ce qu'on opère en présence d'un surfactif stable aux acides et au chlore.